# EUROPÄISCHE PATENTSCHRIFT

(11) **EP 0 956 266 B1**
(45) Veröffentlichungstag und Bekanntmachung des Hinweises auf die Patenterteilung: **13.06.2001**
(21) Anmeldenummer: 97909320.0
(22) Anmeldetag: 26.09.1997
(51) Int. Cl.: C01G 23/00, A61K 7/00, C09C 1/36, C09D 5/32, C03C 8/12

(54) **Ti-Sn-O-NANOCLUSTER, VERFAHREN ZU IHRER HERSTELLUNG UND VERWENDUNG DERSELBEN**
Ti-Sn-O NANOCLUSTERS, METHOD FOR THEIR PRODUCTION AND THEIR USE
NANOCLUSTERS DE Ti-Sn-O, LEUR PROCEDE DE PRODUCTION ET LEUR UTILISATION

(30) Priorität: 27.09.1996 US 714933
(43) Veröffentlichungstag der Anmeldung: 17.11.1999
(73) Patentinhaber: Coty B.V., 1822 BM Alkmaar (NL)
(72) Erfinder: WELLINGHOFF, Stephen, T., San Antonio, TX 78250 (US); CERNASOV, Domnica, Ringwood, NJ 07556 (US)
(74) Vertreter: Walter, Wolf-Jürgen
(86) Internationale Anmeldenummer: EP9705286
(87) Internationale Veröffentlichungsnummer: WO9813299

(56) Entgegenhaltungen:
- EP-A- 0 261 560
- EP-A- 0 499 863
- WO-A-93/22386
- BE-A- 857 288
- US-A- 3 663 284
- US-A- 5 670 583
- PATENT ABSTRACTS OF JAPAN vol. 012, no. 501 (C-556), 27.Dezember 1988 & JP 63 210027 A (TAKI CHEM CO LTD), 31.August 1988,

## Beschreibung

Die vorliegende Erfindung betrifft neue Ti-Su-O-Nanopartikel, diese enthaltende Zusammensetzungen zum Schutz gegen ultraviolette Strahlung sowie Verfahren zur Herstellung derartiger Nanopartikel.

Aus JP-A-63-210027 (Pat. Abstracts of Japan, Vol. 012, No. 501, C-556) ist ein kristallines TiO₂-SnO₂-Sol und dessen Herstellung aus Titaniumtetrachlorid, Zinnchlorid und Wärmebehandlung bei ≥100 °C bekannt.

Die EP-A-499863 beschreibt ein feinteiliges TiO₂ mit SnO₂-Gehalten, wobei die Herstellung aus Titaniumoxidhydrat und Zinnoxid über mehrere Stufen bei verschiedenen pH-Werten und Temperaturen erfolgt und zu einem nadelförmigen TiO₂ mit Rutilstruktur führt.

Die US-A-5372796 beschreibt ein Verfahren zur Herstellung von Metalloxid-Clustern, und in der Zusammenfassung ausgewählter Einladungsunterlagen für das "Internationale Symposium on Advances in Sol-Gel-Processing and Applications" im August 1993 gibt es eine Offenbarung eines Verfahrens, bei dem Oxidpartikel in Nanogröße in einem nichtwäßrigen Medium gezüchtet werden können und die mit Trialkylsiloxangruppen überzogen werden, die zur Beendigung des Wachstums dienen und sichern, daß die Partikel redispergiert werden können, um in vielen Lösungsmitteln klare Lösungen zu bilden. Klare Lösungen von Partikeln in polymerisierbaren Lösungsmitteln unter Einsatz von bis zu 40 Vol-% der unkoagulierten Partikel in Nanogröße können ebenfalls hergestellt werden, und die Polymerisation des Gemisches führt zu transparenten Plaquen und Filmen.

Es wird vermutet, daß Titaniumoxid-Nanocluster eine Oberfläche haben, die im hohen Maße acidisch ist und aus verschiedenen Arten von Lewis-Säure-Stellen besteht. Es ist bekannt, daß auf Titaniumoxid ein solcher Zustand existiert. Das Koordinativpotential von hydroxylierten Titaniumionen ist ebenfalls bekannt, und es werden starke Komplexe mit α-Hydroxycarbonsäuren gebildet, die mit Aminen und Metallhydroxiden neutralisiert werden können. In einigen Fällen können auch Elektronentransferkomplexe mit Elektronendonatoren gebildet werden, wo die Ti⁺⁴-Ionen vollständig zu Ti⁺³-Ionen reduziert werden können.

Während Titaniumoxidpartikel ursprünglich in sehr kleinen Teilchengrößen hergestellt werden können, etwa 50 Å, können sie allerdings nur selten so geliefert werden infolge der Tatsache, daß die Partikel stets im hohen Maße koagulieren. Während weiterhin Titaniumoxid-Nanocluster bei Raumtemperatur in hochsauren Lösungen anscheinend unbegrenzt stabil sind, führt das Erhitzen oder die Verdünnung derartiger Partikel entweder zu einem flockigen Niederschlag oder zu einem elastischen Gel.

Es ist eine Menge Arbeit darauf auf Titaniumoxid basierende Sole und Gele verwendet worden, um die Größe der Nanocluster möglichst wesentlich kleiner als die Wellenlänge des sichtbaren Lichtes zu machen.

Diese Merkmale von Titaniumoxid sind in verschiedenen Arten von Zusammensetzungen, die ultraviolette Strahlung absorbieren, wie Sonnenschutzzusammensetzungen verwendet worden, wo Titaniumdioxid schon seit langem im Gebrauch ist. Ein entscheidender Vorteil besteht darin, daß Titaniumdioxid für die Verwendung im Kosmetika eingesetzt worden ist, und es ist bekannt, daß amorphes Titaniumoxid oder -oxyhydrid kurze Wellenlängen absorbiert. Somit werden für Teilchengrößen von Titaniumoxid, die der Wellenlänge von sichtbarem Licht näherkommen, die hohen Extinktionskoeffizienten des Titaniumdioxids in eine sehr hohe Reflexion für Strahlung umgesetzt, die normalerweise durch Partikel von Nanogröße absorbiert würde. Allerdings werden Titaniumoxid-Nanocluster, die überragende UV-Absorptionseigenschaften haben, infolge ihres festgestellten Mangels an Stabilität in nicht zufriedenstellendem Maße in Sonnenschutzzusammensetzungen verwendet worden.

Gegenwärtig ist es für UV-absorbierende Zusammensetzungen und insbesondere für Sonnenschutzmittel auch bekannt, organische Chromophore für eine Langzeit-UV-Lichtstabilisation von Polymeren zu verwenden. Dazu gehören Salicylate, Benzophenone, Cinnamate, Benzotriazole und ähnliche. Das wichtige molekulare Charakteristikum aller starken UV-Absorptionsmittel ist die Nähe einer Hydroxygruppe an einem Atom X mit einem ungepaarten Elektron, das eine in einer Ebene liegende Bindung X--H--O im angeregten Zustand bilden kann. Somit sind bestimmte Benzophenone, Cinnamate und Salicylate annehmbar für Sonnenschutzmittel oder andere Verwendungen in Kosmetika für den Einsatz im Bereich 200 bis 340 nm. Allerdings haben diese organischen Verbindungen, insbesondere die Cinnamate, eine hautreizende Wirkung, insbesondere bei hohen Konzentrationen, die zur Erreichung hoher Lichtschutzfaktoren (LSF) erforderlich sind, normalerweise bei einem LSF oberhalb von 15. Dieses Problem ist insbesondere akut für Mensch und Tier mit hochempfindlicher Haut oder für solche mit Haut-Displasias oder Melanomen.

Aus diesem Grunde ist der Wunsch entstanden, nicht-organische Sonnenschutzzusammensetzungen zu verwenden, um diese Probleme mit der Empfindlichkeit zu vermeiden. Dieses Problem mit der Sensitivität ist auch vorhanden mit dem neuerdings empfohlenen Sonnenschutzmittel Butyl-methoxydibenzoylmethan, das eine gute Absorption sowohl im UVA- als auch im UVB-Bereich zeigt.

In diesen Sonnenschutzmitteln werden die organischen Chromophoren, wie es üblich ist, mit Feuchthaltemittel auf Lipidbasis oder Okklusionsmitteln und Emulgatoren vermischt, um eine öl-in-Wasser-Emulsion herzustellen, die das Licht streut. Normalerweise arbeitet diese auf folgende Weise: Wenn die Creme auf der Haut als dünne Schicht aufgebracht ist, verdampft das Wasser sehr schnell, und die Schicht wird überwiegend eine Einzelphase mit wesentlich geringerer Lichtstreuung. Die Kraft zur Sonnenblockierung (Absorption und Rückstreuung) dieser Gemische auf der Haut ist eine Funktion der Hautoberflächenrauhigkeit, der Löslichkeit der kosmetischen Grundformulierung in der Haut mit Unterstützung des Emulgators und der Brechungsindexunterschiede zwischen der gequollenen Hautschicht und der darüber liegenden unlöslichen Phase.

Die vorliegende Erfindung betrifft neue stabilisierte Titanium-Zinn-Oxid-Nanopartikel, Verfahren zu deren Herstellung und Zusammensetzungen zum Schutz gegen ultraviolette Strahlung unter Verwendung derselben, wobei Probleme der Hautsensitivität vermieden werden, und die als Hauptpflegezusammensetzungen mit einer angenehmen kosmetischen Erscheinungsform angewandt werden können, und die eine sehr wirksame Absorption von Licht zwischen 200 und 340 nm haben.

Zusammengefaßt betrifft die vorliegende Erfindung einen Nanocluster, stabilisiert gegen Koagulation bei pH größer als 1 in wäßriger Lösung und als Trockenpuder bei Temperaturen bis zu etwa 290° C, bestehend aus einem Ti-Sn-O-Nanocluster, komplexiert mit einem Salz einer α-Hydroxysäure.

Die Erfindung betrifft auch eine Zusammensetzung zum Schutz gegen ultraviolette Strahlung, umfassend einen Träger und einen komplexen Ti-Sn-O-Ladungstransfer-Nanocluster.

Weiterhin betrifft die Erfindung ein Verfahren zur Herstellung eines Ti-Sn-O-Ladungstransfer-Nanoclusterkomplexes durch Hydrolyse eines Titaniumoxids in einer wäßrigen Lösung mit einer Säure und anschließend Umsetzung des hydrolysierten Titaniumoxids mit SnX₂, worin X ein Halogenid ist.
Fig. 1 ist ein Diagramm, das das UV- und sichtbare Spektrum der beiden Pulver von Beispiel 1 zeigt bei Wellenlängen von 280 bis 600 nm;
Fig. 2 ist ein Diagramm, das einen Vergleich des Absorptionsspektrums von mikronisiertem TiO₂ und Ti-Sn-O-Formulierungen bei Weglängen von 15 *µ*m bei Wellenlängen von 280 bis 600 nm zeigt; und
Fig. 3 und 4 sind Diagramme, die die in vitro-Lichtschutzfaktoren der Formulieren A und B von Beispiel 3 zeigen.

Bei dem Verfahren der vorliegenden Erfindung werden stabilisierte Titanium-Zinn-Oxid-Nanocluster zum Zwecke der verstärkten Absorption von UV-Strahlung gebildet. Es ist insbesondere wünschenswert, α-Hydroxysäurekomplexe der Ti-Sn-O-Nanocluster zu bilden, um eine Einphasenlösung bei Volumenfraktionen bis zu 40 % in wäßrigen sowie alkoholischen Lösungen zu bilden. Dies ist sehr nützlich bei der Minimierung der kosmetisch unangenehmen Lichtstreuung, wenn sie auf der Haut als Teil einer topischen Präparation auftritt.

Das Verfahren besteht in erster Linie in der Umsetzung einer Titaniumoxid-Nanoclusterlösung unter sauren Bedingungen mit einem Zinnhalogenid, vorzugsweise einem Chlorid, obgleich Bromid- und Iodidsalze ebenfalls verwendet werden können. Eine beliebige starke anorganische oder organische Säure kann verwendet werden, um die sauren Bedingungen zu schaffen, wobei HCl bevorzugt ist. Jedoch sind auch andere Säuren geeignet, wie HBr, HF, H₂SO₄, CF₃(OOH) und ähnliche. Das SnCl₂ ist vorzugsweise in Lösung in Alkohol oder einer konzentrierten sauren Lösung, vorzugsweise bei einer Konzentration von etwa 0,1 bis 1 M. Die molaren Verhältnisse der Reaktionsteilnehmer sind etwa 0,1 bis 0,3 Sn zu 1 Ti. Diese Reaktionen werden unter Umgebungsbedingungen für eine ausreichende Zeit durchgeführt, um einen Titaniumoxid-Nanoclusterkomplex mit Sn zu bilden. In Abhängigkeit von den Besonderheiten der Reaktionsteilnehmer kann dies bei etwa 5 bis 40° C für 1 Minute bis 1 Stunde variieren, wobei die optimale Reaktionszeit und -temperatur für gegebene Reaktionsteilnehmer durch Routineversuche bestimmt werden können.

Diese Komplexe als solche sind stabil und müssen gegen Koagulierung in Lösung erfindungsgemäß stabilisiert werden durch weitere Reaktion der erhaltenen Ti-Sn-O-Nanocluster mit einem Titaniumalkoxid, vorzugsweise einem der Formel Ti(OR)₄, worin R eine C₃- bis C₄-Alkylgruppe ist, vorzugsweise eine Isopropylgruppe, bei Raumtemperatur (0 bis 40° C) für 1 Minute bis 1 Tag in Methanol-Wasser-Lösungen. Das Verhältnis der Ti-Sn-O-Nanocluster zu dem Ti(OR)₄ beträgt vorzugsweise 1 zu 0,25 bis 0,5. Nach dieser Reaktion wird der erhaltene Reaktionsteilnehmer mit einem Salz einer α-Hydroxysäure komplexiert, wie Citronensäure, Maleinsäure, Milchsäure, Glycolsäure und Weinsäure. Ebenso geeignet sind Oligomere von Polyvinylalkohol oder Polyvinylpyrrolidon mit α-Hydroxysäure-Enden. Ein besonders geeignetes Salz ist Trinatriumcitrat, und dies wird für jedes Mol der Ti-Sn-O-Nanocluster mit 0,25 bis 3 Molen des Trinatriumcitrates in Wasser verwendet. Es ist auch möglich, den pH-Wert durch Zugabe von Alkali, wie Natriumhydroxid, ohne Ausfällung der Nanocluster einzustellen. Dies kann in Wasser-Methanol-Lösungen bei Raumtemperatur (0° bis 40° C) erfolgen.

Die Lösung kann als solche beibehalten werden oder ein Pulver gebildet werden durch Entfernung des Wassers, wie durch Vakuumverdampfung. Das Ergebnis ist ein gelbes Pulver. Dieses stabilisierte Pulver kann wieder in Wasser oder Alkoholen gelöst werden, sogar nach Erhitzen bis zu 290° C für eine halbe Stunde.

Während verschiedene Lösungsmittel verwendet werden können, um das erhaltene Pulver zu lösen, sind auch übliche kosmetische Lösungsmittel, wie Glycerin und Wasser für diesen Zweck geeignet. Es wurde gefunden, daß diese besonders wirksame Lösungsmittel sind, die in der Lage sind, bis zu 40 Gew.-% des Citratkomplexes der Ti-Sn-O-Nanocluster zu lösen.

Es ist auch wünschenswert, das erhaltene Pulver zur Verbesserung von dessen Eigenschaften zu tempern. Insbesondere erhöht das Tempern die Dichte des Pulvers durch Verbesserung der Ti-Sn-O-Ladungstransfer-Wechselwirkung und durch Entfernung der Flüchtigen. Das Tempern erfolgt vorzugsweise in Luft bei 200 bis 280° C für 5 Minuten bis 1 Stunde. Ein besonders vorteilhafter Aspekt des getemperten Ti-Sn-O-Citratkomplexes besteht darin, daß der Extinktionskoeffizient eines solchen Komplexes zwischen 280 bis 340 mn wenigstens dreimal größer ist, bezogen auf die Gesamtkonzentration von Ti-Sn-O, als die des Ti-O-Citratkomplexes. Dieser wesentlich höhere Extinktionskoeffizient pro Gramm des Komplexes für das Ti-Sn-O-Material ist ein besonderer Vorteil bei der Minimierung der Auftragungsdicke der Zusammensetzung, die dieses Material enthält, was für einen hohen LSF für UVA und UVB erforderlich ist.

Diese stabilisierten Ti-Sn-O-Komplex-Nanocluster koagulieren nicht bei einem pH größer als 1 in wäßriger Lösung und als trockenes Pulver bei Temperaturen bis zu etwa 290° C. Sie haben eine Teilchengröße von etwa 20 Å bis 100 Å für eine optimale UV-Absorption. Der Zusammenhang ist gegenwärtig noch nicht ganz klar, jedoch wird vermutet, daß eine Erhöhung des pH während der Reaktion bei höherer Temperatur die Teilchengröße erhöht.

Die erhaltenen Komplexe sind neu und, wie bereits festgestellt, besonders für die Verwendung in Zusammensetzungen als UV-Absorptionsmitteln geeignet.

Während die neuen stabilisierten Nanocluster der vorliegenden Erfindung in einer breiten Vielfalt von Zusammensetzungen verwendet werden können, wo ein Schutz gegen ultraviolette Strahlung erforderlich ist, wie in Möbelpolituren, Sonnenschutzmitteln, Farben, Emails, Plastikgegenständen und anderen bekannten Gegenständen, bei denen UV-Absorptionsmittel verwendet werden, werden sie speziell beschrieben in Zusammenhang mit Sonnenschutzzusammensetzungen für die topische Anwendung. Es ist eine besonders nützliche Eigenschaft der Ti-Sn-O-Citronensäurekomplexe der vorliegenden Erfindung, daß sie entweder als trockenes Pulver oder als 40 %ige Glycerin- oder wäßrige Lösungen emulgiert werden können mit üblichen Erweichungsmitteln, oberflächenaktiven Mitteln Feuchthaltemitteln/Benetzungsmitteln, pH-Regulierern, Dicken- oder Filmbildnern, Sonnenschutzfiltern, Parfümen, Farbstoffen, Gelatisierungsmitteln, Schutzmitteln und ähnlichen, die üblicherweise vorhanden sind, um alkoholische oder wäßrige Sonnenschutzzusammensetzungen mit unterschiedlichen Lichtschutzfaktor herzustellen. Spezielle Beispiele dieser Additive sind unten aufgeführt.
Erweichungsmittel:
   Cetylester, Cetyllactat, Cetylpalmitat, Maisöl, Diisopropyl Adipate, Diisopropyl Dimer Dilinolate, Traubenkernöl, hydriertes Rizinusöl, hydriertes Kokosnußöl, hydriertes Sojabohnenöl, Isopropylpalmitat, Isopropylmyristat, Lauryllactat, Maleinsäure-verestertes Sojabohnenöl, Octyldodecanol, Octylisononanoat-Ether, Natriumhyaluronat, lösliches Collagen, Squalen, Sterolester und ähnliche.
Oberflächenaktive Mittel:
   Cetearylalkohol, Cetylalkohol, DEA-cetylphosphat, Disodium Laureth Sulfosuccinate, Glycol Distearate, Laneth-40, Lauryl Lactate, Magnesium Lauryl Sulphate, Oleth-3, PEG-2 Diisononanoate, PEG-150, PEG-15 Cocamine, PEG-40 hydrogenated Castor Oil, PEG-8 Laurate, PEG-20 Stearate, Polysorbate 20, PPG-4 Myristyl Ether Acetate, Sorbitan Laurate, Sorbitan Stearate, Stearate-10 und ähnliche.
Feuchthaltemittel/Benetzungsmittel:
   Glycerin, Butylenglycol, Propylenglycol, Natriumhyaluronat, Aloe vera, Glycose, Glycereth-26, Glycereth-7 Triacetate, Milchsäure, Lactose, PEG-6, PEG-32 und ähnliche.
pH-Regulatoren:
   Triethanolamin, Natriumhydroxid, Ammoniumhydroxid, Citronensäure, Dinatriumphosphat, Glycolsäure, Kaliumhydroxid, Amminomethylpropanol und ähnliche.
Verdickungsmittel/Filmbildner:
   Acrylic/Acrylate Copolymer, Carbomer 934, Carbomer 941, Hydroxypropylcellulose, Hydroxypropylmethylcellulose, Xanthangummi, Magnesiumaluminiumsilicat und ähnliche.
Sonnenschutzfilter:
   Octyl Methoxycinnamate, Octyl Dimethyl PABA, Benzophenone-1, Benzophenone-3, Benzophenone-9, Drometrizole, Octyl Salicylate, Phenylbenzimidazol-5-sulfonsäure und ähnliche.

Oft wird auch gewünscht, Farbstoffe und Parfüme in derartige Zusammensetzungen einzubeziehen. Derartige und andere Additive, die oben aufgeführt worden sind, können in die Sonnenschutzzusammensetzungen in ihren üblichen Mengen und mit den üblichen Wirkungen einbezogen werden.

Die Erfindung wird im Zusammenhang mit den folgenden Beispielen beschrieben, wobei diese nur der Erläuterung dienen.

### Beispiel 1

Auf Titanium basierende Nanocluster wurden hergestellt durch Vermischen von 25 ml (8,4 x 10⁻² Mol) Titaniumisopropoxid mit 100 ml 1M HCl (0,1 Mol HCl; 5,56 Mol H₂O), um zu einer anfänglich trüben Lösung zu gelangen, die sich innerhalb von 2 Stunden klärte. Nach Alterung von 2 Stunden bis 1 Tag wurden Versuche unternommen, diese Lösung bis zur Trockne bei 80° C im Rotationsverdampfer zu verdampfen. Es bildete sich allerdings ein weißes Koagulat, das auch in konzentrierter HCl unlöslich war. Es wurde auch beobachtet, daß die Fällung bei Raumtemperatur für pH größer als 1 auftreten würde.

Da die sehr aktiven Oberflächenhydroxyle an der Oberfläche der Titaniumoxid-Nanocluster für die Fusion der Teilchen verantwortlich waren, wurden diese Gruppen mit dem Trinatriumcitrat-Dihydrat (Na₃C) neutralisiert und komplexiert.

1,3 g Titaniumoxid-Nanocluster wurden in 2 ml Wasser gelöst, und es wurde tropfenweise 1,5 g Trinatriumcitrat-Dihydrat (Na₃C), gelöst in 3 ml Wasser, hinzugegeben, was ausreichend war, um den pH-Wert auf 4 zu erhöhen. Die Zugabe von 3,0 g Na₃C erhöhte den pH-Wert auf 7. Nach Trocknung unter Vakuum bei 80 bis 90° C konnte das erhaltene weiße Pulver auf 270° C ohne Unlöslichmachung erhitzt werden, was ein Anzeichen dafür war, daß das an der Oberfläche vorhandene Ti-OH wirksam komplexiert worden war.

Das ultraviolette und sichtbare Spektrum einer Wasserlösung mit einem 1:1 Gewichtsverhältnis von Clustern zu Na₃-citrat, das bei 200° C in Luft für mehrere Stunden getempert wurde, zeigt ein kontinuierliches Ansteigen des Extinktionskoeffizienten von 360 nm bis 200 nm infolge des Lösungsmittelverlustes. Citrat hat nur eine kleine Einflußgröße auf die Absorption um etwa 210 bis 200 mm. Der gemessene Extinktionskoeffizient, bezogen auf das Gewicht des Titaniumoxidclusters, beträgt 300 nm-5600 cm⁻¹ und 320 nm-2300 cm⁻¹.

### Beispiel 2

Ein Titaniumoxid-Nanocluster-Ladungstransferkomplex mit Sn in Wasser wurde hergestellt durch Vermischen von 25 ml (8,4 x 10⁻² Mol) Titaniumisopropoxid mit 100 ml 1M HCl (0,1 Mol HCl; 5,56 Mol H₂O), um zu einer anfänglich trüben Lösung zu gelangen, die sich innerhalb von zwei Stunden klärte. Nach Alterung über zwei Stunden bis 1 Tag wurden 3,125 g SnCl₂ in 20 ml MeOH (1,63 x 10⁻² Mol), das filtriert worden war, unter Rühren zu der nichtkonzentrierten Titanium-Nanoclusterlösung gegeben. Der erhaltene gelbe Farbton war ein Anzeichen für den Ti-Sn-O-Oberflächenkomplex.

Andere mit Verfahren der Zinnzugabe wurden versucht, um die Menge des dem Gemisch zugegebenen Wassers, das später im Verfahren zu verdampfen wäre, zu minimieren. Dies schloß ein das Lösen von 3,33 x 10⁻³ Molen SnCl₂ in 0,826 ml konzentrierter HCl (3,47 x 10⁻² Mol H₂O; 9,91 x 10⁻³ Mol HCl) und anschließend Zugabe von 8,4 x 10⁻³ Mol Titaniumisopropoxid. Es trat eine sehr leuchtende Orangefarbe auf, die sich nach mehreren Sekunden in ein helleres Orange umwandelte.

Nach der anfänglichen Zugabe von 1 ml Wasser zu diesem Gemisch bildete sich ein gelber Niederschlag. Allerdings war die Zugabe von zwei weiteren ml Wasser ausreichend, um eine klar gelbe Lösung herzustellen. Die sichtbare und UV-Absorption dieser Lösung konnte erhöht werden durch Erhitzen einer verdünnten Version dieser Lösung auf 70° C, wo eine Fällung auftrat.

Da ein Niederschlag in diesen Lösungen über einen Zeitraum von mehreren Tagen auftrat, wurde die obige Prozedur wiederholt, mit Ausnahme dessen, daß die zusätzliche Stufe der Zugabe von zusätzlichen 0,7 x 10⁻² Mol Titaniumisopropoxid zu dem Gemisch genutzt wurde, um das an der Oberfläche chemisorbierte Sn abzukapseln, das für die Phasentrennung verantwortlich war. Diese Behandlung führte zu einer klaren gelben Lösung nach etwa 2 Minuten, die unbegrenzt klar blieb.

Obgleich die Lösung von diesen Dreischicht-Nanoclustern bis zur Trockenheit bei 50 bis 60° C gepumpt werden konnte, um ein sehr acidisches gelbes Pulver (Ti-Sn-O-) zu bilden, das in Wasser wieder gelöst werden konnte, machte das Erhitzen auf 200° C für eine Zeit von nur 10 bis 20 Minuten das Pulver unlöslich.

Wiederum wurden mit den Einkomponenten-Titan-Nanoclustern die aktiven Oberflächengruppen mit Na₃C komplexiert, um die Acidität zu neutralisieren und die Cluster thermisch zu stabilisieren. Die Lösung wurde im Vakuum bei 90° C bis zu einem trockenen gelben Pulver verdampft, das in Wasser und Glycerin leicht löslich war bei Gewichtsfraktionen bis zu 40 %.

Es war nicht notwendig, die Ti-Sn-O-Lösung mit Na₃C vollständig zu neutralisieren. Bei einer typischen Verfahrensweise wurden 0,771 g Ti-Sn-O in Wasser gelöst, mit 0,385 g Na₃C in Wasser gemischt, mit 1 N NaOH-Lösung (1,5 ml) auf pH = 5 neutralisiert und bis zur Trockne unter Vakuum eingedampft. Das auf diese Weise hergestellte gelbe Pulver konnte bei 270° C für 15 bis 30 Minuten in Luft getempert werden und sowohl in Wasser als auch im Glycerin wieder gelöst werden.

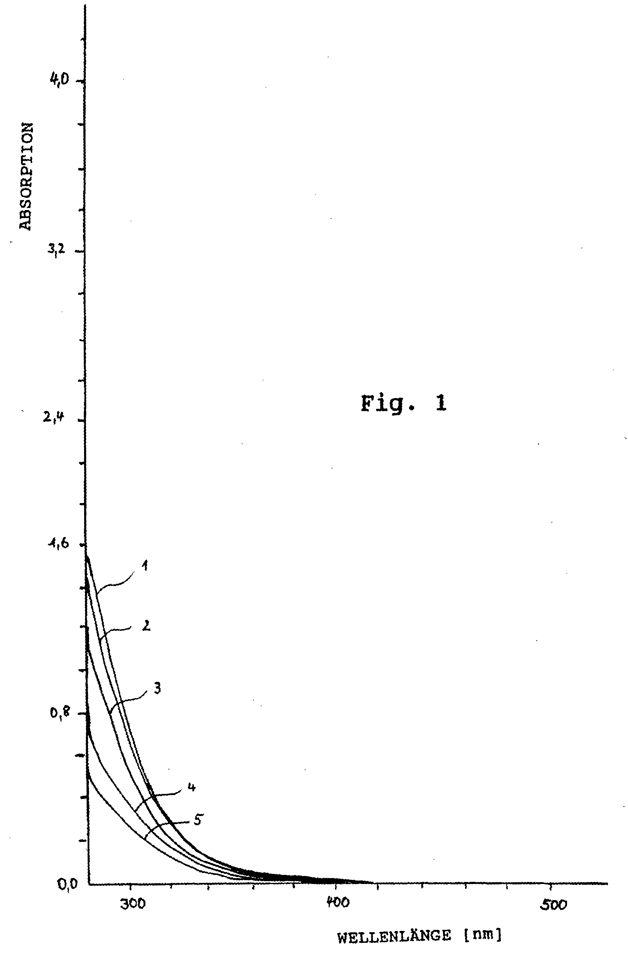

Das UV und das sichtbare Spektrum der beiden Pulver, jeweils gelöst in einer 5 % wäßrigen Lösung und verschiedene Mengen an Ti-Sn-O und Na₃C enthaltend, neutralisiert mit Natriumhydroxid auf pH=5, wurde aus einer 15 *µ*m Gießfolie zwischen zwei Quarzplatten erhalten. Es tritt eine wesentliche Verschiebung im Spektrum in Richtung zum sichtbaren Bereich auf durch Komplexierung der Titaniumoxid-Nanopartikel mit Sn, wie in Fig. 1 gezeigt.

Wie erwartet besaß das Pulver mit den höchsten Ti-Sn-O-Gehalt und der höchsten Dichte, gesichert durch Tempern bei 270° C in Luft, den höchsten Extinktionskoeffizienten in Nähe des UV-Bereiches. Die Extinktionskoeffizienten (cm⁻¹) für Lösungen 1:1 TiO₂:Na₃C, bezogen auf das Gewicht, und 58 Gew-% TiO₂ in Wasser gemäß Fig. 1 werden in Tabelle I verglichen.

**Tabelle I**

| | 280 nm | 300 nm | 320 nm |
|---|---|---|---|
| TiO₂ | 13.560 | 5600 | 2300 |
| Ti-Sn-O | 31.840 | 17.910 | 6900 |
| 270° C (getempert) | | | |

Bei einer Zusammensetzung 58 Gew-% TiO₂, 9 Gew-% SnCl₂, 11 Gew-% Na(+), 22 Gew-% Citrat wurde getempert
- Kurve 1:: zusätzlich bei 270 °C für 5 Minuten
- Kurve 2 :: zusätzlich bei 200 °C für 10 Minuten
- Kurve 3:: bei 110 °C für 15 Minuten.

Bei einer Zusammensetzung
24 Gew-% TiO₂, 11 Gew-% SnCl₂, 15 Gew-% Na(+), 50 Gew-% Citrat wurde getempert
- Kurve 4:: bei 200 °C für 15 Minuten
- Kurve 5 :: bei 270 °C zusätzlich für 15 Minuten.

### Beispiel 3

Zwei kosmetische Wasser-in-öl-Formulieren (A und B) wurden mit unterschiedlichen Beladungen des Ti-Sn-O-Komplexes, 3 Gew-% (A) und 5 Gew-% (B) hergestellt. Die Ti-Sn-O-Komplexe wurden hergestellt durch Verdünnen von 40 Gewichtsteilen des Komplexes mit 60 Gewichtsteilen Glycerin und 60 Gewichtsteilen Wasser. Das Gemisch wurde bei 100° C für etwa 1 Stunde zum Sieden gebracht bis alle Feststoffe (der Komplex) gelöst waren und das Wasser verdampft war. Man erhielt eine dicke, gelbliche, klare Lösung. Für die Formulierung A (3 Gew-% reines Ti-Sn-O) wurden 7,5 Gew-% mit den Komponenten, die unten aufgeführt sind, einbezogen, und für die Formulierung B (5 Gew-% reines Ti-Sn-O) waren es 12,5 Gew-%.

Die Komponenten sind in Tabelle II aufgeführt, wo die Anteile in Gewichts-% angegeben sind.

**Tabelle II**

| | A | B |
|---|---|---|
| Cetyl Dimethicone Copolyol | 2,50 | 2,50 |
| Octyl Palmitate | 4,00 | 4,00 |
| Octyl Stearate | 1,50 | 1,50 |
| Cetyl Dimethicone | 1,00 | 1,00 |
| hydriertes Rizinusöl | 0,50 | 0,50 |
| mikrokristallines Wachs | 1,00 | 1,00 |
| Cyclomethicone | 7,50 | 7,50 |
| Diisopropyl Dimer Dilinoleate | 2,00 | 2,00 |
| Octyldodecyl Neopentanoate | 9,50 | 7,50 |
| Ti-Sn-O/Glycerin | 7,50 | 12,50 |
| dest. Wasser | 61,75 | 58,70 |
| NaCl | 0,60 | 0,60 |
| Phenoxyethanol | 0,50 | 0,50 |
| Parfüm (Pritania 33133T) | 0,15 | 0,20 |

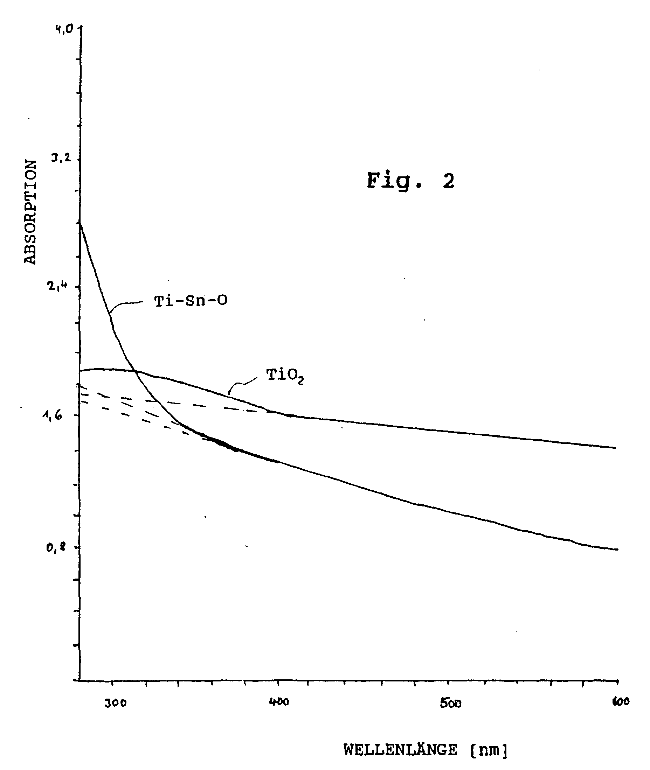

Fig. 2 ist ein Vergleich zwischen einem 5 Gew-% mikronisierten TiO₂, formuliert in einer kosmetischen Grundlage, und einem 5 Gew-% 3:1 Ti-Sn-O : Na₃C (Formulierung "B" oben), der zuerst in Glycerin mit 40 Gew-% gelöst wurde und anschließend in einer kosmetischen Grundlage dispergiert wurde. Die Absorptionsfähigkeit des Ti-Sn-O-Gemisches ist deutlich wesentlich höher unterhalb 320 nm, jedoch die ziemlich frequenzunabhängige (nichtdispersive) Streuung des mikronisierten TiO₂-Gemisches liegt deutlich über 320 nm.

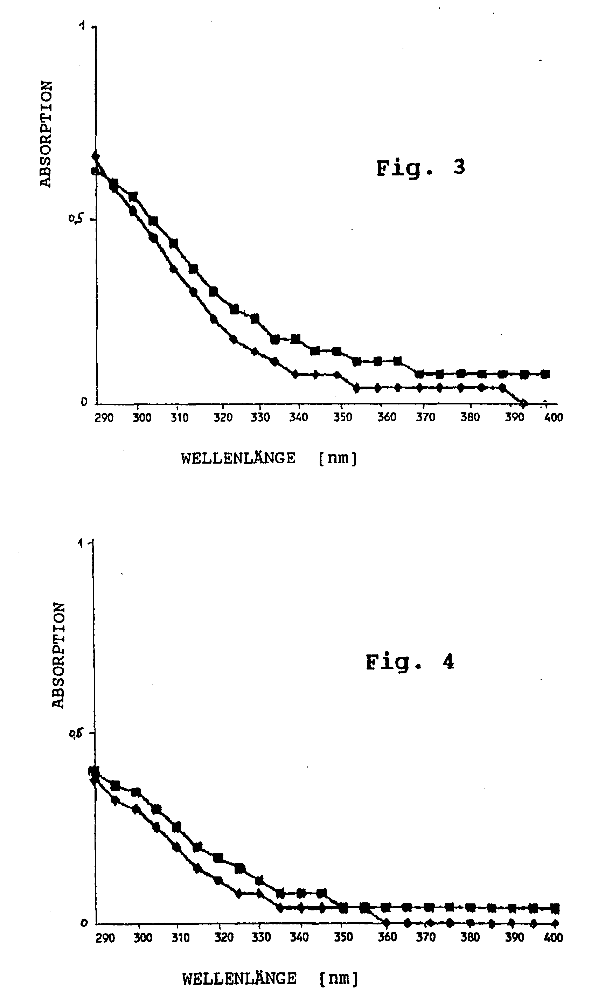

Fig. 3 und 4 zeigen die in vitro-LSF (Lichtschutzfaktor)-Berechnungen für die Formulierungen A und B. Die Werte sind nachfolgend tabellarisch wiedergegeben:
zu Fig. 3:

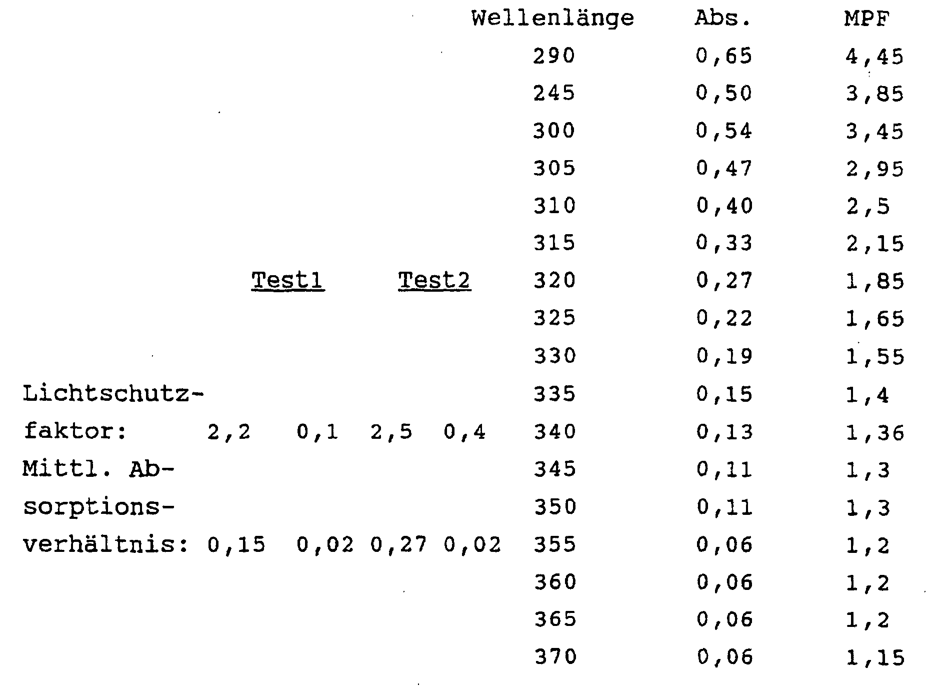

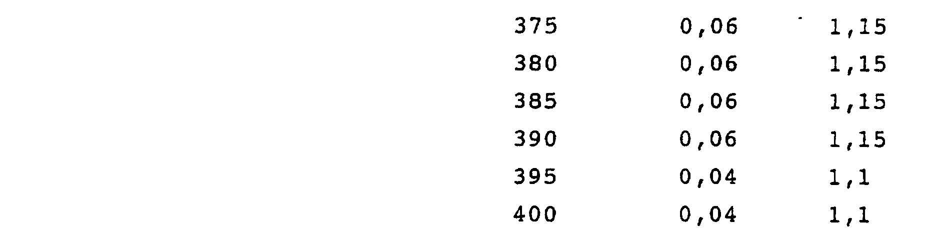

zu Fig. 4

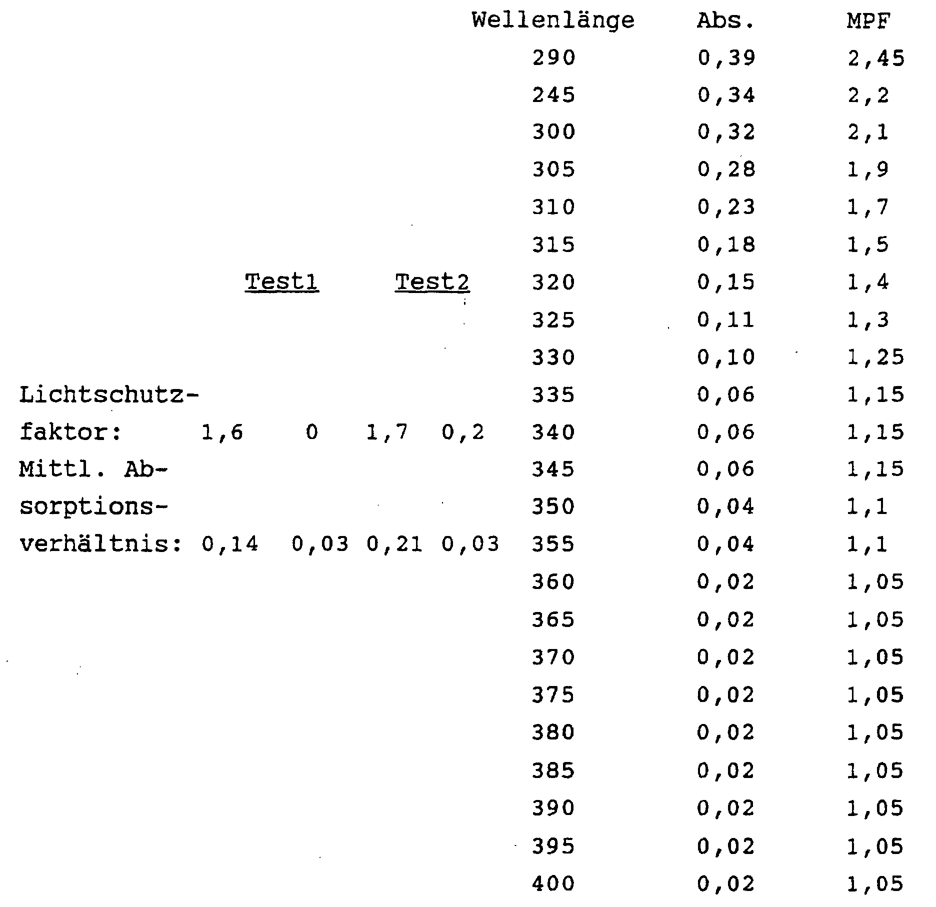

Es ist ersichtlich, daß bei der Herstellung von Zusammensetzungen für die topische Anwendung zum Schutz gegen ultraviolette Strahlung, die Menge an zugegebenen Nanoclustern zum Träger in breitem Maße variieren kann in Abhängigkeit vom Grad des gewünschten Schutzes (LFS). Die spezielle Menge an zugegebenen Nanoclustern, um den gewünschten LSF für einen gegebenen Träger zu ergeben, kann durch Routineversuche unter Heranziehung der zuvor genannten Beispiele und der LSF-Berechnungen für einen beispielhaften Träger unter Variierung der Mengen an Nanoclustern bestimmt werden.

Die erfindungsgemäße Beschreibung und die bevorzugten Beispiele beschränken den Schutzumfang der Erfindung nicht.

## Patentansprüche

1. Ti-Sn-O-Nanocluster, bestehend aus einem Ti-Sn-O-Nanocluster, der mit einem Salz einer α-Hydroxysäure komplexiert ist und der stabilisiert ist gegen Koagulierung bei einem pH-Wert von größer als 1 in wäßriger Lösung und der stabilisiert ist als ein trockenes Pulver bis zu Temperaturen von 290° C.

2. Nanocluster nach Anspruch 1, worin der komplexierte Nanocluster getempert ist.

3. Nanocluster nach Anspruch 1 oder 2, worin die Säure Citronensäure ist.

4. Nanocluster nach Anspruch 1 oder 2, worin das Salz Trinatriumcitrat ist.

5. Zusammensetzung zum Schutz gegen ultraviolette Strahlung, umfassend einen Träger und Ti-Sn-O-Ladungstransfer-Nanoclusterkomplexe in einer wirksamen Menge, um ultraviolette Strahlung zu absorbieren.

6. Zusammensetzung nach Anspruch 5, worin die Nanoclusterkomplexe getempert sind.

7. Zusammensetzung nach Anspruch 5 oder 6, worin die Nanocluster in einer Menge von wenigstens etwa 3 Gew-% für jeweils 100 Gew-% der Zusammensetzung vorhanden sind.

8. Zusammensetzung nach Anspruch 5 oder 6, worin die Nanocluster Ti-Sn-O-Nanocluster, komplexiert mit einem Salz einer α-Hydroxysäure, umfassen.

9. Zusammensetzung nach Anspruch 5 oder 6, worin die Nanocluster Ti-Sn-O-Nanocluster, komplexiert mit Trinatriumcitrat umfassen.

10. Verfahren zur Herstellung von Ti-Sn-O-LadungstransferNanoclusterkomplexen, umfassend das Hydrolysieren eines Titaniumalkoxids in einer wäßrigen Lösung mit einer Säure und anschließend Umsetzung des hydrolysierten Titaniumalkoxids mit SnX₂, worin X ein Halogenid ist, bei einer Temperatur von 5 bis 40 °C für 1 Minute bis 1 Stunde bis zur Bildung der Nanocluster, und anschließende Komplexierung mit einem Salz einer α-Hydroxysäure.

11. Verfahren nach Anspruch 10, gekennzeichnet durch die Stufe der Stabilisierung der Nanocluster vor der Komplexierung mit dem Salz der α-Hydroxysäure mit einem Titaniumalkoxid.

12. Verfahren nach Anspruch 10, gekennzeichnet durch die Stufe der Erhöhung der Ladungstransferwechselwirkung der komplexierten Nanocluster durch Tempern derselben bei 200 bis 280 C für 5 Minuten bis 1 Stunde.

13. Verwendung von Ti-Sn-O-Nanoclustern, komplexiert mit einem Salz einer α-Hydroxysäure, als UV-Absorptionsmittel in kosmetischen Formulierungen in einem Anteil von 1 bis 15 Gew-% neben üblichen kosmetischen Wirk- und Trägerstoffen.

14. Verwendung von Ti-Sn-O-Nanoclustern, komplexiert mit einem Salz einer α-Hydroxysäure, als UV-Absorptionsmittel in Farben, Emails oder Kunststoffen.

## Claims

1. A Ti-Sn-O nanocluster comprising a Ti-Sn-O nanocluster which is complexed with a salt of an alpha-hydroxy acid and which is stabilized against coagulation at pH>1 in aqueous solution and which is stabilized as a dry powder at temperatures up to 290°C .

2. The nanocluster of Claim 1 wherein said complexed nanocluster is annealed.

3. The nanocluster of Claim 1 or 2 wherein said acid is citric acid.

4. The nanocluster of Claim 1 or 2 wherein said salt is trisodium citrate.

5. A composition for protection against ultraviolet radiation comprising a carrier and Ti-O-Sn charge transfer complex nanoclusters in an amount effective to absorb ultraviolet radiation.

6. The composition of Claim 5 wherein said complex nanoclusters are annealed.

7. The composition of Claim 5 or 6 wherein said nanoclusters are present in an amount of at least about 3 wt. % for each 100 wt. % said composition.

8. The composition of Claim 5 or 6 wherein said nanoclusters comprise Ti-Sn-O nanoclusters complexed with a salt of an alpha-hydroxy acid.

9. The composition of Claim 5 or 6 wherein said nanoclusters comprise Ti-Sn-O nanoclusters complexed with trisodium citrate.

10. The method of making Ti-O-Sn charge transfer complex nanoclusters comprising hydrolyzing a titanium alkoxide in an aqueous solution with an acid and then reacting the hydrolyzed titanium alkoxide with SnX₂, in which X is a halide, at a temperature of 5 to 40 °C for 1 minute to 1 hour sufficient to form said nanoclusters and after that stabilizing said nanoclusters by complexing them with a salt of an alpha-hydroxy acid.

11. The method of Claim 10, including the step of stabilizing said nanoclusters with a titanium alkoxide before complexing them with a salt of an alpha-hydroxy acid.

12. The method of Claim 10, including the step of increasing the charge transfer interaction of said complexed nanoclusters by annealing the same at 200 to 280 °C for 5 minutes to 1 hour.

13. Use of Ti-Sn-O nanoclusters complexed with a salt of a alpha-hydroxy acid as a UV radiation absorber in cosmetic formulations with a share of 1 to 15 wt. % apart from usual cosmetically effective and supporting components.

14. Use of Ti-Sn-O nanoclusters complexed with a salt of a alpha-hydroxy acid as a UV radiation absorber in paints, enamels and plastic articles.

## Revendications

1. Nanocluster de Ti-Sn-O, comprenant un nanocluster de Ti-Sn-O, qui est complexé avec un sel d'un acide à-hydroxylique et qui est stabilisé par rapport à la coagulation avec un pH supérieur à 1 en solution aqueuse et qui est stabilisé comme une poudre sèche jusqu'à des températures de 290 °C.

2. Nanocluster selon la revendication 1, dans lequel le nanocluster complexé est malléabilisé.

3. Nanocluster selon la revendication 1 ou 2, dans lequel l'acide est de l'acide citrique.

4. Nanocluster selon la revendication 1 ou 2, dans lequel le sel est du citrate trisodique.

5. Composé pour la protection contre le rayonnement ultraviolet, comprenant un support et des complexes de nanoclusters à transfert de charge Ti-Sn-O dans une quantité efficace pour absorber le rayonnement ultraviolet.

6. Composé selon la revendication 5, dans lequel les complexes de nanoclusters sont malléabilisés.

7. Composition selon la revendication 5 ou 6, dans laquelle les nanoclusters sont présents dans une quantité d'au moins environ 3 % de poids pour respectivement 100 % de poids de la composition.

8. Composition selon la revendication 5 ou 6, les nanoclusters comprenant des nanoclusters de Ti-Sn-O complexés avec un sel d'un acide á-hydroxylique.

9. Composition selon la revendication 5 ou 6, les nanoclusters comprenant des nanoclusters de Ti-Sn-O complexés avec du citrate trisodique.

10. Procédé pour la fabrication de complexes de nanoclusters à transfert de charge Ti-Sn-O comprenant l'hydrolisation d'un alcoxyde de titanium dans une solution aqueuse avec un acide suivie de la transformation de l'alcoxyde de titanium hydrolysé avec du SnX₂, X étant un halogénure, à une température de 5 à 40 °C pour 1 minute jusqu'à 1 heure jusqu'à la formation des nanoclusters, et complexation consécutive avec un sel d'un acide à-hydroxylique.

11. Procédé selon la revendication 10, caractérisé par le niveau de la stabilisation des nanoclusters avant la complexation avec le sel de l'acide á-hydroxylique avec un alcoxyde de titanium.

12. Procédé selon la revendication 10, caractérisé par le niveau de l'élévation de l'interaction de transfert de charge des nanoclusters complexés par la malléabilisation de ceux-ci de 200 jusqu'à 280 °C pour une durée de 5 minutes à 1 heure.

13. Utilisation de nanoclusters de Ti-Sn-O, complexés avec un sel d'un acide á-hydroxylique, comme absorbant de UV dans des formulations cosmétiques dans une fraction de 1 à 15 % de poids parallèlement aux agents actifs et agents supports cosmétiques classiques.

14. Utilisation de nanoclusters de Ti-Sn-O, complexés avec un sel d'un acide á-hydroxylique, comme absorbant de UV dans des teintures, des émaux ou des plastiques.
